# EUROPEAN PATENT SPECIFICATION

(11) **EP 4 385 438 B1**
(45) Date of publication and mention of the grant of the patent: **04.11.2026**
(21) Application number: 22855120.6
(22) Date of filing: 01.07.2022
(51) Int. Cl.: A61B 18/14, A61B 18/12, G06T 17/00, A61B 34/20, A61B 17/00, A61B 18/00, A61B 5/01, A61B 5/024, A61B 5/367, A61B 34/10, A61B 5/0215

(54) **ABLATION SYSTEM**
ABLATIONSSYSTEM
SYSTÈME D'ABLATION

(30) Priority: 11.08.2021 CN 202110916536; 11.08.2021 CN 202111509147
(43) Date of publication of application: 19.06.2024
(73) Proprietor: Shanghai Microport Ep Medtech Co., Ltd., Shanghai 201318 (CN)
(72) Inventor: PENG, Yahui, Shanghai 201318 (CN); SUN, Yiyong, Shanghai 201318 (CN); SHEN, Liuping, Shanghai 201318 (CN); YU, Zhili, Shanghai 201318 (CN); WANG, Xinyi, Shanghai 201318 (CN); HE, Jialiang, Shanghai 201318 (CN)
(74) Representative: Arnold & Siedsma
(86) International application number: PCT/CN2022/103388
(87) International publication number: WO 2023/016136

(56) References cited:
- WO-A1-2019/217430
- CN-A- 103 385 705
- CN-A- 105 125 279
- CN-A- 110 198 680
- CN-A- 112 336 445
- CN-A- 112 336 445
- CN-A- 112 842 514
- CN-A- 113 349 923
- US-A1- 2004 078 036
- US-A1- 2004 127 894
- US-A1- 2015 342 662
- US-A1- 2016 095 651
- US-A1- 2016 128 770

## Description

### TECHNICAL FIELD

The present invention relates to an ablation system.

### BACKGROUND

With the development of minimally invasive intervention technology, nerve ablation is increasingly used clinically, which is mainly applied to treat symptoms such as hypertension, diabetes, heart disease, cancer and tumors, and has achieved great results.

However, the inventors of the present application realize that the distribution of sympathetic nerves in the renal artery varies from person to person, and it is difficult to determine whether the target tissue region to be ablated contains the sympathetic nerves. Accordingly, at present, the ablation location is usually randomly selected. In such a manner, the ablation may be repeated or missed, eventually leading to ablation failure. Furthermore, in conventional technology, the ablation catheter is controlled and the ablation site is positioned with the help of X-ray imaging. The X-ray imaging is a two-dimensional image and is very unclear, resulting in inaccurate positioning of the ablation catheter and inability to perform accurate ablation, so that the ablation effect is unsatisfactory. US2016128770A1 discloses an electronic control unit configured to acquire a value for an ablation description parameter and/or a position signal metric, wherein the value corresponds to a location in the tissue. The electronic control unit is further configured to evaluate the value, assign it a visual indicator of a visualization scheme associated with the parameter/metric corresponding to the value, and generate a marker comprising the visual indicator such that the marker is indicative of the acquired value. US2004127894A1 relates to control for an ablation catheter that provides increased ablation catheter operation feedback and increased options for programmable control of the ablation catheter for clinicians. The control for an ablation catheter has a microcontroller containing memory, an input and output coupled to the microcontroller, a movement program, a comparison program, and an annunciating program.
In CN113349923A , a method for performing a medical procedure is disclosed, including bringing a probe into contact with an organ in a body of a patient. A map of the organ is displayed, and the location of the probe relative to the map is tracked. A therapy is applied via the probe at multiple tissue sites in the organ with which the probe is brought into contact. Stability of the contact between the probe and the tissue sites is assessed while applying the therapy. The map is automatically marked, responsively to the assessed stability, to indicate the tissue sites at which the therapy was applied.

### SUMMARY

According to an embodiment of the present invention, an ablation system according to claim 1 is provided.

The above-mentioned ablation system includes the information processing module, and the signal input and output module in communication with each other. The signal input and output module is configured to acquire the spatial position information of the ablation site according to the current position of the medical catheter, and transmit the spatial position information of the ablation site to the information processing module; the information processing module is configured to acquire the target spatial position information according to the target ablation site, and generate the ablation prompt information according to the target spatial position information of the trarget ablation site and the spatial position information of the ablation site, thereby preventing the position of the medical catheter from deviating from the target ablation site during the ablation, causing problems such as repeated ablation or ineffective ablation, accordingly, the accurate ablation is guaranteed.

The details of one or more embodiments of the present invention are set forth in the accompanying drawings and the description below.

### BRIEF DESCRIPTION OF THE DRAWINGS

In order to describe the technical solution in the embodiments of the present invention more clearly, accompanying drawings required for the description of the embodiments will be briefly introduced. Obviously, the accompanying drawings in the following description are merely some of the embodiments of the present invention, and those skilled in the art can obtain other drawings according to these drawings without any inventive effort.
FIG. 1 is a structural block diagram of an ablation system according to one or more embodiments.
FIG. 2 is a schematic structure diagram of an ablation system in practical application according to one or more embodiments.
FIG. 3 is a schematic diagram of functional modules of the ablation system according to the embodiment shown in FIG. 2.
FIG. 4 is a schematic diagram illustrating renal artery ablation according to one or more embodiments, in which a three-dimensional geometric model and ablation site markers displayed by a display module are shown in a dashed box.
FIG. 5 is a workflow chart of an ablation system according to one or more embodiments.

100, three-dimensional mapping system; 101, working module; 102, signal control unit; 103, signal receiving unit; 200, excitation field generator; 300, ablation instrument; 301, physiological parameter sensor; 302, neutral electrode; 303, medical catheter; 401, right kidney; 402, abdominal aorta; 403, right renal artery; 404, left renal artery.

### DETAILED DESCRIPTION OF THE EMBODIMENTS

In order to make the technical solution and advantages of the present invention clearer, the present invention will be further elaborated below with reference to the drawings and embodiments. It should be appreciated that the specific embodiments described here are merely used for explaination of the present invention, rather than limiting the present invention. In the present invention, "proximal end" and "distal end" refer to the relative orientation, relative position, and direction of elements or actions with respect each other from the perspective of a physician using the product, although "proximal end" and "distal end" are not restrictive, the "proximal end" generally refers to an end of the product closest to the physician during a normal operation, while the "distal end" or "head end" generally refers to one end that first enters the patient's body.

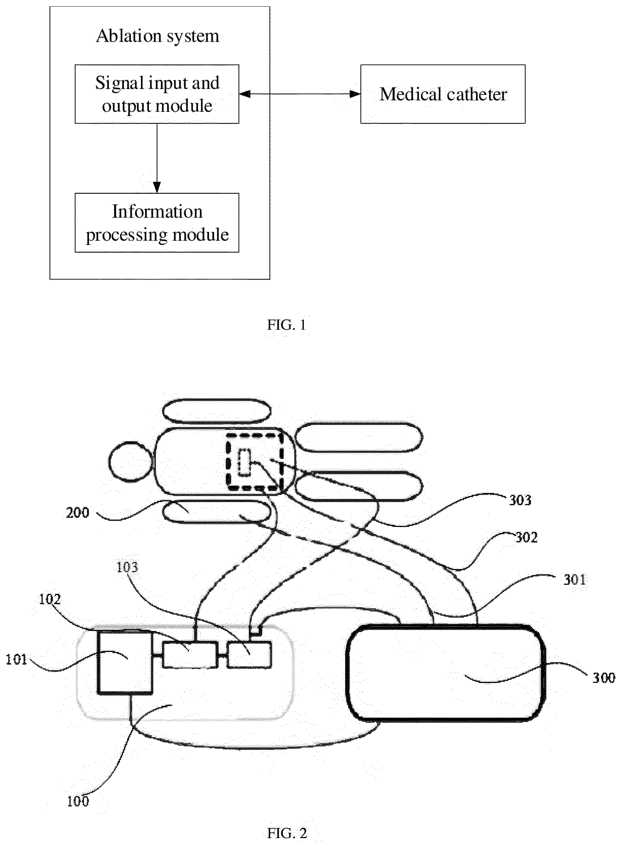

In some embodiments, as shown in FIG. 1, the present application provides an ablation system, which includes an information processing module and a signal input and output module in communication with each other. The signal input and output module is configured to be connected to at least one medical catheter 303, and acquire spatial position information of an ablation site according to a current position of the medical catheter 303 when the medical catheter 303 outputs ablation energy, and transmit the spatial position information of the ablation site to the information processing module. The information processing module is configured to acquire target spatial position information according to a target ablation site, periodically calculate and obtain to-be-compared position information according to the spatial position information of the ablation site within a predetermined time period, and periodically compare the to-be-compared position information to the target spatial position information to generate ablation prompt information.

The target ablation site may be a recommended ablation site, such as a recommended ablation site recommended after physiological stimulation (the recommended ablation site will be described in detail below), or a target ablation site determined by other means, such as a target ablation site determined by a doctor according to the ablation situation. The spatial position information of the ablation site is spatial position information of an electrode on the medical catheter 303 that performs the ablation. During the ablation, the information processing module compares the spatial position information of the ablation site acquired in real time to the target spatial position information, to generate the ablation prompt information. The ablation prompt information may be outputted through a display mode and/or voice mode, etc., to provide the user with a prompt.

For example, the ablation prompt information may be outputted through the voice mode. In some embodiments, different types of speaking templates are preset. When the spatial position information of the ablation site and the target spatial position information satisfy certain requirements, the information processing module acquires and outputs a corresponding speaking template.

The ablation prompt information may also be outputted through the display mode. In some embodiments, the signal input and output module is further configured to acquire modeling spatial position information according to spatial position information of a distal end of the medical catheter 303, and transmit the modeling spatial position information to the information processing module. The information processing module is further configured to construct a three-dimensional geometric model of a region where the distal end of the medical catheter 303 is located according to the modeling spatial position information, and mark the generated ablation prompt information in the three-dimensional geometric model. Optionally, the three-dimensional geometric model is displayed on the display module. The display module and the construction of the three-dimensional geometric model will be detailed below.

In the above embodiment, the spatial position information of the ablation site refers to the spatial position information of the distal end of the medical catheter 303 when the medical catheter 303 outputs the ablation energy. Specifically, the spatial position information of the ablation site refers to the spatial position information of the ablation electrode at the distal end of the medical catheter 303 when the medical catheter 303 outputs the ablation energy. The predetermined time period may be preset, such as one second, and in other embodiments, the predetermined time period may be other values. The step that the to-be-compared position information is calculated and obtained according to the spatial position information of the ablation site within the predetermined time period may include that the to-be-compared position information is obtained by calculating statistical information of the spatial position information of all ablation sites within the predetermined time period, for example, taking an average, that is, average position information of the ablation electrode of the medical catheter 303 within the time period, such as an average position coordinate of the ablation electrode within one second. Alternatively, it is also possible to remove spatial position information of some ablation sites that are obviously wrong within the predetermined time period through preliminary screening, and obtain the to-be-compared position information by calculating the statistical information of spatial position information of the remaining ablation sites within the predetermined time period. Alternatively, it is also possible to calculate statistical information of spatial position information of some representative ablation sites selected within the predetermined time period to obtain the to-be-compared position information. A first preset distance is also set in advance, such as 1 mm. In other embodiments, the first preset distance may be other values.

It should be noted that the ablation prompt information may include at least one of identification information and operation prompt information of each position in a region to be ablated. The identification information of each position may include, but is not limited to, recommended ablation site information, unrecommended ablation site information, or information indicating whether each recommended ablation site is ablated. The operation prompt information is configured to prompt whether spatial position information of a current ablation site satisfies an operation requirement according to the target spatial position information. The operation prompt information may include, but is not limited to, prompt information for ablation position movement, prompt information for repeated ablation, etc.

The above-mentioned ablation system includes the information processing module and the signal input and output module communicating with each other; through the signal, the signal input and output module acquires the spatial position information of the ablation site according to the current position of the medical catheter 303, and transmits the spatial position information of the ablation site to the information processing module; the information processing module is configured to acquire the target spatial position information according to a target ablation site, and generate the ablation prompt information according to the target spatial position information of the target ablation site and the spatial position information of the ablation site, which can prevent the medical catheter 303 from deviating from the target ablation site during the ablation and causing problems such as repeated ablation or ineffective ablation, thereby ensuring accurate positioning of the distal end of the medical catheter 303 and ensuring accurate ablation.

In some embodiments, referring to FIG. 1, the signal input and output module may be connected to the medical catheter 303 and configured to acquire the spatial position information of the ablation site according to the current position of the medical catheter 303. Specifically, the spatial position information of the ablation site refers to the spatial position information of the distal end of the medical catheter 303 when the medical catheter 303 outputs ablation energy. More specifically, the medical catheter 303 is provided with an electrode which is configured to perform stimulation, ablation or positioning, and so on. The spatial position information of the ablation site refers to spatial position information corresponding to an ablation electrode provided on the medical catheter 303 when the medical catheter 303 outputs the ablation energy.

In the above embodiments, the medical catheter 303 may include one or more electrodes to output energy through the one or more electrodes. In addition, it should be noted that the ablation system may further include a neutral electrode 302, or the ablation system is connected to a neutral electrode 302. The neutral electrode 302 serves as an energy loop. The distal end of the medical catheter 303 is provided with a spatial position information collection device that communicates with the signal input and output module. The spatial position information collection device may be a three-dimensional positioning sensor, such as a magnetic positioning sensor. Accordingly, the distal end of the medical catheter 303 can be positioned in real time through the spatial position information collection device. Of course, the electrode provided on the medical catheter 303 itself can also serve as the three-dimensional positioning sensor to acquire the spatial position information, which will not be limited herein.

Specifically, in practical applications, the ablation system may include a three-dimensional mapping system 100 and a radio frequency ablation instrument 300. The three-dimensional mapping system 100 and the radiofrequency ablation instrument 300 may share a processor, or the three-dimensional mapping system 100 and the radiofrequency ablation instrument are respectively provided with processors. The processor of the three-dimensional mapping system 100 is in communication with the processor of the radiofrequency ablation instrument 300 to perform the information transmission. The processor described here is the information processing module in the embodiment. That is to say, the information processing module in the embodiment may be shared by the three-dimensional mapping system 100 and the radiofrequency ablation instrument 300. Alternatively, the information processing module may include two portions, one portion is located in the three-dimensional mapping system 100, the other portion is located in the radiofrequency ablation instrument 300, and the two portions can communicate with each other. In other embodiments, the three-dimensional mapping system 100 and the radiofrequency ablation instrument 300 may be integrated into one in the ablation system, and accordingly, one information processing module can be adopted to process relevant data.

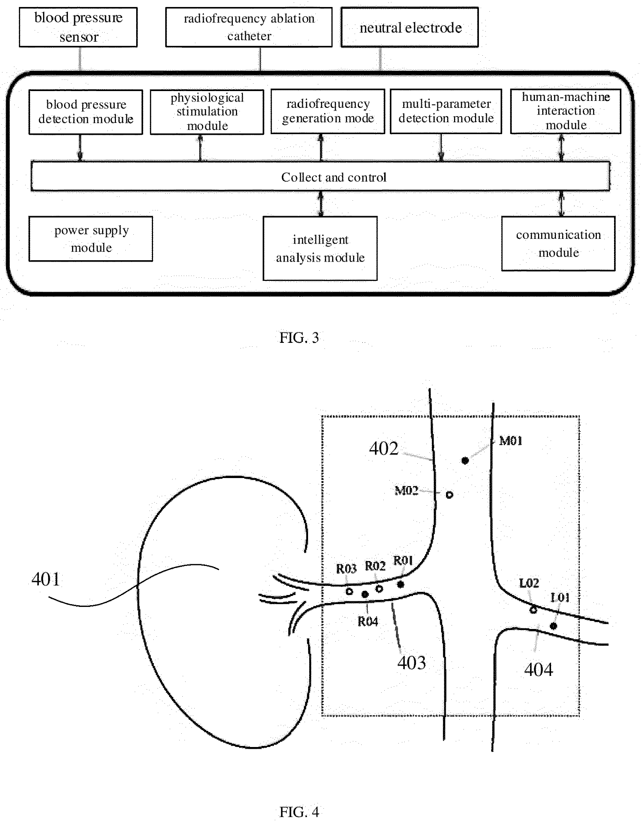

With reference to FIGS. 2 and 3, in the embodiment, the case where the three-dimensional mapping system 100 and the radiofrequency ablation device 300 are respectively provided with processors is taken as an example for description. The information processing module includes a working module 101 provided in the three-dimensional mapping system 100 in FIG. 2 and an intelligent analysis module provided in the radiofrequency ablation instrument 300 in FIG. 3; the working module 101 and the intelligent analysis module can communicate with each other. The medical catheter 303 is an ablation catheter connected to the radiofrequency ablation instrument 300. The medical catheter 303 is connected to the signal input and output module of the three-dimensional mapping system 100 to implement the configuration of the system. Specifically, the embodiments shown in FIGS. 2 and 3 are taken for illustration, the signal input and output module of the three-dimensional mapping system 100 may include a signal receiving unit 103 and a signal control unit 102. A portion of the information processing module is the working module 101 in the FIG. 2, while the other portion is the intelligent analysis module in the FIG. 3.

As shown in FIG. 2, when in use, an excitation field generator 200 of the three-dimensional mapping system 100 is first installed. The excitation field generator 200 is configured to output an excitation field for three-dimensional positioning. Optionally, the excitation field generator 200 is placed under a hospital bed, for example, installed at a position under the hospital bed corresponding to a location on the patient that requires ablation. Taking the renal artery as an example, the excitation field generator 200 is placed at a position under the hospital bed adjacent to the patient's waist. The excitation field generator 200 may be a magnetic field, an electric field or other energy field generators, and the signal outputted by the excitation field generator 200 is a currently known harmless, low-power, medium-low frequency electric field or magnetic field signal, and thereby the spatial position information can be acquired through the magnetic field positioning, electric field positioning, or impedance positioning imaging technologies.

The signal control unit 102 controls the output of the excitation field, so that the signal receiving unit 103 can receive the spatial position information collected by the spatial position information collection device of the distal end of the medical catheter 303 when the distal end of the medical catheter 303 enters an effective range of the excitation field, and transmit the spatial position information to the signal control unit 102. Optionally, the spatial position information received by the signal receiving unit 103 is an analog signal, so that the signal receiving unit 103 modulates, demodulates and digitizes the analog signal before transmitting the analog signal to the signal control unit 102. The signal control unit 102 processes and calculates the spatial position information to obtain three-dimensional positioning data including a three-dimensional coordinate, a direction, an angle and other information. Accordingly, the signal processing module can acquire the spatial position information from the signal control unit 102 to perform the three-dimensional modeling and/or image processing, or to generate the ablation prompt information together with other information, etc. In other embodiments, the working module 101 may be further configured to establish a three-dimensional geometric model of a region in which the distal end of the medical catheter 303 is located according to the spatial position information, and record the generated ablation prompt information into the three-dimensional geometric model for subsequent data recording and analysis.

For the above-mentioned ablation system, the working module 101 in the information processing module is further configured to record spatial position information of an ablated site. Specifically, when the medical catheter 303 outputs ablation energy, the working module 101 in the information processing module periodically calculates to-be-compared position information according to the spatial position information of the ablation site within a predetermined time period, periodically compares the to-be-compared position information to the target spatial position information, and record the target spatial position information as the spatial position information of the ablated site when the medical catheter 303 completes the ablation if a distance calculated according to the current to-be-compared position information within the current predetermined time period and the target spatial position information is less than or equal to a first preset distance.

Furthermore, the working module 101 in the information processing module may be further configured to monitor whether the ablation position moves. Specifically, the work module 101 in the information processing module is configured to periodically calculate the to-be-compared position information according to the spatial position information of the ablation site within the predetermined time period when the medical catheter 303 outputs the ablation energy, and periodically compare the to-be-compared position information to the target spatial position information. When the distance, calculated according to the current to-be-compared position information within the current predetermined time period and the target spatial position information, is less than or equal to the first preset distance, the information processing module outputs a prompt indicating that the ablation position moves.

In other embodiments, the working module 101 in the information processing module can be selectively configured to record the spatial position information of the ablation site or monitor whether the ablation position moves, and these functions are not required to be present simultaneously. In some embodiments, the working module 101 or the signal control unit 102 may transmit the spatial position information of the ablation site and the target spatial position information to the intelligent analysis module in the ablation instrument 300, and the intelligent analysis module completes the function of recording the spatial position information of the ablated site or monitoring whether the ablation position moves, which will not be limited herein.

The working module 101 or the intelligent analysis module in the information processing module may be further configured to compare the current to-be-compared position information to the target spatial position information to determine whether the current to-be-compared position information and the target spatial position information correspond to the same ablation site.

When the distance, calculated according to the current to-be-compared position information within the current predetermined time period and the target spatial position information, is less than or equal to the first preset distance, it means that the distance change is within a certain range, and when the medical catheter 303 completes the ablation, the target spatial position information is recorded as the spatial position information of an ablated site. It should be noted that the spatial position information of the ablated site may refer to spatial position information of an ablation site at which ablation duration is finished or a qualified ablation effect index is reached, or may refer to spatial position information of an ablation sitewhere a doctor believes the ablation is completed, which will not be limited herein. The completion of the ablation may refer to that an ablation duration set by the system is finished, or the ablation duration set by the system is up, or may refer to that a user (e.g., doctor) determines that the ablation is completed according to experience, which will not be limited herein.

When the distance, calculated according to the current to-be-compared position information within the current predetermined time period and the target spatial position information, is greater than the first preset distance, it means that the distance changes beyond the certain range, and a prompt indicating that the ablation position moves is presented. Specifically, the information processing module, when determining whether the ablation position moves, may transmit the prompt information or alarm information to the display module, so that the prompt information or the alarm information can be issued through the display module. In other embodiments, the prompt indicating that the ablation position moves can also be outputted in the form of voice prompt.

In practical applications, the working module 101 or the intelligent analysis module in the information processing module calculates an average position coordinate of the ablation electrode in each period of time during the ablation in real time, such as calculating the average position coordinate of the ablation electrode every 1 second and storing it. The average position coordinate is compared to the target spatial position information. When the distance changes within a certain range (such as 1mm), it is considered to be the same ablation site, and when the distance changes beyond the certain range, the user (doctor) is reminded that the ablation position moves. The doctor can view the ablation effect index of the position corresponding to the target spatial position information and determine the ablation situation at the target ablation site, or the doctor can determine according to the experience whether to continue the ablation at the position or whether the ablation site needs to be re-marked, etc. The prompt information or alarm information indicating that the ablation position moves is transmitted to the display module via a communication module to issue the prompt information or alarm information through the display module. The communication module will be described in detail below.

In the above embodiment, by monitoring the displacement between the ablation electrode and a target tissue during the ablation, the movement of the electrode in the ablation process is prevented from affecting the ablation effect.

In the above embodiment, the information processing module is further configured to compare the current to-be-compared position information within the current predetermined time period to spatial position information of any previously ablated site, and when the distance, calculated according to the current to-be-compared position information within the current predetermined time period and target spatial position information of any previously ablated site, is less than a second preset distance, the information processing module outputs a prompt indicating repeated ablation.

Specifically, the second preset distance is also set in advance, such as 2 mm. In other embodiments, the second preset distance may be other values. It is determined whether the distance, calculated according to the current to-be-compared position information within the current predetermined time period and the target spatial position information of any previously ablated site, is less than the second preset distance, and if yes, the information processing module outputs a prompt indicating repeated ablation and it is determined whether the new ablation site needs to be repeatedly ablated according to the user's selection. The new ablation site refers to the current position to be compared within the current predetermined time period.

Optionally, after the information processing module outputs the prompt indicating the repeated ablation, the user can determine whether to continue the ablation at the new ablation site. When the user determines to continue the ablation at the new ablation site, position information of the new ablation site serves as the target spatial position information, and the to-be-compared position information continues to be periodically calculated according to the spatial position information of the ablation site within the predetermined time period in the ablation process, and the above processing continues.

In the above embodiment, by monitoring the displacement between the ablation electrode and the target tissue during the ablation, ineffective ablation caused by the electrode movement in the process of the ablation is avoided.

In the above embodiments, by calculating the change in the position coordinate of the ablation electrode during the ablation, the displacement of the ablation electrode during the ablation is monitored and a prompt or alarm is issued to avoid ineffective ablation.

The above-mentioned ablation system may further include a physiological stimulation module and a physiological parameter detection module. In the embodiment, the physiological stimulation module and the physiological parameter detection module are provided in the ablation instrument 300. The physiological parameter detection module is configured to collect a physiological parameter through a physiological parameter sensor 301. The physiological stimulation module is connected to the medical catheter 303. The physiological stimulation module is configured to generate a stimulation signal according to a stimulation parameter, so that the medical catheter 303 outputs the stimulation energy. The physiological parameter detection module is configured to detect the physiological parameter generated in response to the stimulation signal, and transmit the physiological parameter to the information processing module. When the medical catheter 303 outputs the stimulation energy, the information input and output module acquires the spatial position information of a stimulation site according to the current position of the medical catheter 303, and transmits the spatial position information of the stimulation site to the information processing module. The information processing module is further configured to generate ablation prompt information according to the physiological parameter and the spatial position information of the stimulation site. The ablation prompt information may include recommended ablation site information and unrecommended ablation site information.

Specifically, the physiological stimulation module is configured to output a physiological stimulation signal which is generally a low-power energy stimulation signal. Optionally, the set stimulation parameter can be inputted through a human-machine interaction module, such as the display module, and then the physiological stimulation module generates the physiological stimulation signal according to the stimulation parameter, and outputs the stimulation energy through the electrode at the distal end of the medical catheter 303, so that the stimulation site on the patient receives the stimulation signal and produces a corresponding physiological response.

The physiological parameter detection module is configured to detect a physiological parameter generated under a stimulation signal. The physiological parameter detection module may be at least one of a blood pressure, heartbeat, body temperature or bioelectric activity. Optionally, the physiological parameter detection module may be a blood pressure detection module. The blood pressure detection module is connected to a blood pressure sensor to detect the blood pressure of the patient. The blood pressure sensor may include a blood pressure monitoring sensor and a supporting accessory. In the embodiment, an invasive blood pressure monitoring mode can be used, or a non-invasive blood pressure monitoring mode can be used instead. When the invasive blood pressure monitoring mode is used, for the renal artery, for instance, the radial artery puncture can be selected, a puncture site is at the same level as the heart and is fixed, and the blood pressure is monitored and recorded in real time. In other embodiments, the physiological parameter detection module can be replaced by other physiological parameter monitoring units that can reflect sympathetic nerve excitation, such as heart rate, body temperature, bioelectrical activity, etc. The body temperature may be collected by a temperature sensor installed at the distal end of the medical catheter 303.

When the medical catheter 303 outputs the stimulation energy, the physiological parameter detection module collects and processes the physiological parameter to obtain a measurement result, and transmits the measurement result to the intelligent analysis module of the information processing module. The intelligent analysis module generates the ablation prompt information according to the physiological parameter and the spatial position information of the stimulation site. The ablation prompt information may include recommended ablation site information and unrecommended ablation site information. Specifically, the intelligent analysis module generates the recommended ablation site information and the unrecommended ablation site information according to the physiological parameter during the stimulation, thereby avoiding repeated physiological stimulation or ineffective ablation at the unrecommended ablation site, and also avoiding missing effective ablation at the recommended ablation site.

Of course, in another embodiment, the intelligent analysis module or the physiological parameter detection module can also transmit the physiological parameter and the spatial position information of the stimulation site to the working module 101 of the three-dimensional mapping system 100, and the working module 101 generates the ablation prompt information, which is not be limited herein.

In some embodiments, the information processing module is further configured to determine the spatial position information of the corresponding stimulation site in the recommended ablation site information as the target spatial position information of the target ablation site, and calculate a distance between the target spatial position information of the current target ablation site and the spatial position information of any one of the previously ablated sites. When the distance is less than or equal to a third preset distance, a prompt indicating repeated ablation is outputted. Specifically, in the above embodiment, the recommended ablation site information may be obtained through analysis by the information processing module, for example, by analysis of the physiological parameter at the stimulation site. When the physiological parameter satisfies the requirement, it means that the stimulation site satisfies the ablation requirement, and is the recommended ablation site. The spatial position information of the stimulation site at the moment, i.e., the spatial position information of the recommended ablation site serves as the target spatial position information of the target ablation site, in which the target spatial position information is also position information of a target site. The prompt indicating repeated ablation is outputted when a distance between two ablation sites (the currently recommended ablation site and the ablated site) is less than or equal to the third preset distance. Each time after the currently recommended ablation site is determined according to the physiological parameter, the information processing module calculates the distance between the current target ablation site, i.e., the currently recommended ablation site and any one ablated site, and compares the distance to the third preset distance. When the distance is less than or equal to the third preset distance, the currently recommended ablation site is determined as a repeated ablation site.

The third preset distance may be set in advance, for example, it can be 2 mm. In other embodiments, the third preset distance may be other values. Preferably, the third preset distance may be the same as the second preset distance, because the second preset distance is a threshold for determining whether ablation sites are the same ablation site. Accordingly, the standards can be unified, and the consistency of determination by the system can be improved.

In the above embodiment, when the currently recommended ablation site is within a certain distance range (e.g., 2mm) of an existing ablation site, the operator is reminded to avoid repeated ablation. By comparing the coordinate of the current ablation electrode to the coordinate of the existing ablation site, repeated ablation at the same position or too close positions can be avoided.

In some embodiments, the working module 101 or the intelligent analysis module in the information processing module is further configured to determine the spatial position information of the corresponding stimulation site in the recommended ablation site information as the target spatial position information of the target ablation site, and calculate the distance between the target spatial position information of the current target ablation site and the to-be-compared position information. When the distance is greater than a fourth preset distance, a prompt indicating that the ablation position moves is outputted.

Specifically, the movement of the ablation position may be caused by a deviation of the medical catheter 303 during the ablation at the target ablation site, or the deviation of the medical catheter 303 from the target ablation site before the ablation. In the embodiment, when the medical catheter 303 deviates from the target ablation site after starting to output the ablation energy, a prompt indicating that the ablation position moves is provided.

Alternatively, the fourth preset distance may be equal to the first preset distance.

Further, the signal input and output module in the above-mentioned ablation system is further configured to acquire the modeling spatial position information according to the spatial position information of the distal end of the medical catheter 303, and transmit the modeling spatial position information to the working module 101 or intelligent analysis module in the information processing module. The information processing module is further configured to construct a three-dimensional geometric model of a region in which the distal end of the medical catheter 303 is located according to the modeling spatial position information, and mark generated ablation prompt information in the three-dimensional geometric model.

Specifically, the established three-dimensional geometric model is an accurate model of a position to be ablated, which is obtained by sending the medical catheter 303 to the position to be ablated before the ablation or during the ablation. When the distal end of the medical catheter 303 enters the effective range, the signal input and output module can receive the modeling spatial position information, so that the information processing module can start modeling. Optionally, in order to ensure the accuracy of the trajectory of the medical catheter 303, the information processing module may store a reference three-dimensional model pre-generated by a medical imaging device, so that the direction of the medical catheter 303 can be determined according to the reference three-dimensional model, and the reference three-dimensional model is mutually referenced with the three-dimensional geometric model generated in real time to improve the accuracy. Accordingly, in the actual processing, the information processing module can acquire the reference three-dimensional model, and then push the medical catheter 303 to a preset position to be ablated according to the reference three-dimensional model. When the distal end of the medical catheter 303 enters the effective range, the signal input and output module may receive the modeling spatial position information and transmit the modeling spatial position information to the information processing module, so that the information processing module can start modeling. During the modeling, the information processing module may use the reference three-dimensional model as a reference to determine whether there exists an unmodeled region and correct position information, etc.

The establishment of the three-dimensional geometric model of a renal artery, for instance, is now described. The medical catheter 303 can enter the human body from the femoral artery vessel of the right leg. The distal end of the medical catheter 303 advances upward along the femoral artery vessel and enters the abdominal aorta 402. At the same time, when the distal end of the medical catheter 303 enters the effective magnetic field excitation range, the signal input and output module can acquire the spatial position information of the distal end of the medical catheter 303. At the moment, it is possible to start building the three-dimensional geometric model of this segment of the arterial blood vessel. When it is observed that the distal end of the medical catheter 303 is adjacent to a branch position of the renal artery in the reference three-dimensional model, the distal end of the medical catheter 303 is controlled to enter the right (or left) renal artery, and meanwhile, a three-dimensional model of the right (or left) renal artery vessel is established. Alternatively, instead of using the reference three-dimensional model, when it is observed that the distal end of the medical catheter 303 is adjacent to the branch position of the renal artery in an X-ray image, the distal end of the medical catheter 303 is controlled to enter the right (or left) renal artery, and meanwhile, the three-dimensional model of the right (or left) renal artery blood vessel is established.

Optionally, the three-dimensional geometric model may include a renal artery vessel and/or an abdominal aorta 402 segment vessel. In order to establish a more accurate three-dimensional model of the blood vessel, the medical catheter 303 can be controlled to move along the blood vessel wall around the region in which the renal artery branch is located, to cover as many sites as possible. The three-dimensional modeling range can be selected to cover the parts adjacent to the left and right renal artery branches of the abdominal aorta 402 (i.e., the proximal and distal ends of the abdominal aorta 402) to better reflect the structural information of this region. Since a main trunk of renal sympathetic nerves is distributed on the adventitia of the abdominal aorta 402, if a target site with concentrated distribution of sympathetic nerves is found, a better ablation effect may be achieved. In such a manner, the three-dimensional positioning and modeling of the vessels extending from the renal artery vessel to its adjacent abdominal aorta 402 segment vessel and the subsequent positioning and ablation of the sympathetic nerve ablation target site based on this result a better ablation effect.

The signal input and output module transmits the modeling spatial position information to the working module 101 or the intelligent analysis module in the information processing module. The working module 101 or the intelligent analysis module is configured to establish the three-dimensional geometric model according to the modeling spatial position information, and generate the ablation prompt information according to the target spatial position information corresponding to the target ablation site and the spatial position information of the ablation site. The ablation prompt information may include information indicating whether a stimulation site determined according to the physiological stimulation is a recommended ablation site or an unrecommended ablation site, and one or more types of ablation information generated by performing the ablation processing at the target ablation site, such as the ablated site, the movement of the ablation position, repeated ablation, etc. The ablation information of the ablated site may include, but is not limited to, an ablation parameter and ablation result information. The ablation parameter is a parameter corresponding to the energy outputted by the medical catheter 303. The ablation result information is evaluation information of the ablation effect after the ablation site is ablated.

Optionally, in some embodiments, the information processing module displays the recommended ablation site information and the unrecommended ablation site information on the three-dimensional geometric model. Further, the information processing module displays the recommended ablation site information and the unrecommended ablation site information in the three-dimensional geometric model as a recommended ablation site marker and an unrecommended ablation site marker.

Specifically, referring to FIGS. 2 and 3, the physiological stimulation module in FIG. 3 serves as the physiological stimulation module in the embodiment, and a multi-parameter detection module and a blood pressure detection module in FIG. 3 serve as the physiological parameter detection module in the embodiment.

In the embodiment shown in FIG. 3 where the renal artery is taken as an example, a distal electrode of the medical catheter 303 enters a renal artery, and the ablation electrode is attached to the blood vessel wall and fixed in position. The information processing module simultaneously marks the position coordinate, and displays the position coordinate with a designated marker on the three-dimensional geometric model. After that, a stimulation parameter is set in a corresponding window on the user interface of the display module, and a certain amount of physiological stimulation pulses is outputted to the renal artery vessel wall. The information processing module determines whether the blood vessel wall includes the sympathetic nerve (or parasympathetic nerves, vagus nerves) according to the physiological parameter during the stimulation, such as a change pattern of the blood pressure, and records the determination result. If the determination result indicates that there only exists the sympathetic nerve or the sympathetic nerve dominates, the position is determined as the recommended ablation site. If it is determined that the blood vessel wall excludes the sympathetic nerve or the sympathetic nerve is not dominant, the position is determined as an unrecommended ablation site. Optionally, the information processing module displays the recommended ablation site information and the unrecommended ablation site information on the three-dimensional geometric model.

In some embodiments, the ablation system may further include an ablation energy module and a display module. The ablation energy module is connected to the medical catheter 303, and the display module is in communication with the information processing module. The ablation energy module is configured to generate an ablation signal according to the ablation parameter, so that the medical catheter 303 outputs the ablation energy. The display module is configured to display the three-dimensional geometric model and the ablation prompt information.

When the medical catheter 303 outputs the ablation energy, the signal input and output module acquires the spatial position information of the ablation site according to the current position of the medical catheter 303, and transmits the spatial position information of the ablation site to the information processing module. The information processing module is configured to acquire target spatial position information according to a target ablation site, and generate ablation prompt information according to the target spatial position information and the spatial position information of the ablation site. The ablation prompt information includes an ablated site marker, and the ablated site marker is displayed on the three-dimensional geometric model.

Specifically, the ablation energy module is mainly configured to output the ablation energy through, for example, a radiofrequency generation mode, and accordingly, the ablation energy is outputted through the medical catheter 303. The ablation energy can be measured by an ablation power or an ablation current. Optionally, the display module can be a human-machine interaction display module, so that an ablation energy parameter can be set through the display module, and accordingly, the ablation energy module can generate corresponding ablation energy according to the ablation energy parameter. When the medical catheter 303 outputs the ablation energy, the information processing module generates the ablation prompt information according to the target spatial position information and the spatial position information of the ablation site. That is, the information processing module is configured to acquire the target spatial position information according to the target ablation site, periodically calculate the to-be-compared position information according to the spatial position information of the ablation site within the predetermined time period, and periodically compare the to-be-compared position information to the target spatial position information to generate the ablation prompt information which may be, for example, an ablated site marker, and display the ablated site marker at a corresponding position on the three-dimensional geometric model.

In the above embodiment, the number of display modules can be set as required, and the content displayed by the display module can be preset according to user habits and the like. Optionally, there may exist one display module, and the display module can be in communication with the information processing module to display all information that needs to be displayed. In other embodiments, there may exist two display modules, one of which is located in the three-dimensional mapping system 100, and the other is located in the radiofrequency ablation instrument 300. The display contents of the two display modules can be set as required. The contents displayed by two display modules can be completely same, or partially same, or completely different. Taking two display modules as an example, one of the display modules is in communication with the working module 101 to display position-related information, and the other display module is in communication with the intelligent analysis module to display ablation-related information. In other embodiments, the display module in communication with the working module 101 may further display the ablation-related information, and the display module in communication with the intelligent analysis module may further display the position-related information. As shown in FIG. 3, FIG. 3 is a schematic structure diagram of an ablation instrument 300 according to an embodiment. In the embodiment, a radio frequency generating module in the ablation instrument 300 is the ablation energy module in the embodiment, and the human-machine interaction module is the display module. The ablation instrument 300 communicates with the working module 101 of the three-dimensional mapping system 100 through a communication module to transmit the ablation-related information to the working module 101. A power supply module of the ablation instrument 300 is configured to power the ablation instrument 300, so that all parts of the ablation instrument 300 can operate normally.

In practical applications, the intelligent analysis module may be configured to process the ablation-related information, and the working module 101 may be configured to process the position-related information. In other embodiments, the types of information processed by the intelligent analysis module and the working module 101 can be preset, which are not specifically limited here. The intelligent analysis module acquires the parameter of the ablation energy through the human-machine interaction module, so that the intelligent analysis module can calculate the ablation information according to the parameter of the ablation energy to control the radiofrequency generation module to generate the ablation energy and output the ablation energy to the medical catheter 303 which in turn ablates the ablation site. During the ablation, the working module 101 displays the acquired spatial position information of the ablation site in the three-dimensional geometric model, and communicates with the ablation instrument 300 through the communication module of the ablation instrument 300, and generates the ablation prompt information according to the target spatial position information and the spatial position information of the ablation site. That is, the information processing module is configured to acquire the target spatial position information according to a target ablation site, periodically calculate the to-be-compared position information according to the spatial position information of the ablation site within the predetermined time period, and periodically compare the to-be-compared position information to the target spatial position information to generate the ablation prompt information, and display the ablation prompt information in the three-dimensional geometric model. In the above embodiment, the ablation prompt information may be generated by the intelligent analysis module according to the target spatial position information and the spatial position information of the ablation site, for example, when the physiological parameter information at the target spatial position (that is, at the target site) satisfies the requirement, an ablated site marker is generated, so that the working module 101 displays the ablated site marker in the three-dimensional geometric model to complete one ablation. In other embodiments, the intelligent analysis module may transmit the physiological parameter information at the target spatial position (i.e., at the target site) to the working module 101, and the working module 101 generates the ablated site marker when determining that the physiological parameter information satisfies the requirement and displays the ablated site marker in the three-dimensional geometric model to complete one ablation.

The renal artery is taken as an example again, when the recommended ablation site serves as the target ablation site, the ablation electrode is closely attached to the vessel wall at the recommended ablation site. The ablation parameter is set in the corresponding window on the user interface of the display module and the ablation is started, that is, the intelligent analysis module can calculate the ablation information according to the parameter of the ablation energy, so that the radiofrequency generation module is controlled to generate the ablation energy and output the ablation energy to the medical catheter 303 for ablation of the ablation site. During the ablation period, the intelligent analysis module acquires the physiological information of the recommended ablation site in real time, such as blood pressure information, to determine whether the ablation at this ablation site satisfies the requirement. When it is determined that the ablation site satisfies the ablation requirement, the ablation may be stopped, otherwise, the ablation needs to be continued. During the ablation, the ablation-related information corresponding to position sites is recorded. After the processing of one position site is completed, the medical catheter 303 is manipulated to allow the ablation electrode to reach other positions on the renal artery wall, and the above process is repeated. Generally, a number of ablation sites need to be processed on a renal artery on one side to ensure the blockade of the sympathetic nerve on this renal artery. The absence of regions that cause blood pressure elevation upon stimulation on that side can serve as the basis for determining the effectiveness of the blockade. After the ablation of the renal artery on this side is completed, the medical catheter 303 is controlled to allow the ablation electrode to enter a renal artery on the other side, and the same process is followed.

In the above embodiment, the ablation energy is generated by the ablation energy module to perform the ablation, and the information processing module displays the generated ablation prompt information in the corresponding three-dimensional geometric model, so that the user can view the relevant ablation prompt information in the three-dimensional geometric model, which is more intelligent, improves the user experience, reduces the operator's learning curve, and improves the effectiveness and safety of the surgery.

In some embodiments, the ablation prompt information may include marker color information. Ablation prompt information of different marker types corresponds to different marker color information.

Specifically, different position sites in the three-dimensional geometric model are distinguished by using different types of markers, for example, color information. As shown in FIG. 4, for example, the recommended ablation site at which the sympathetic nerve is dominant after the physiological stimulation is marked with a yellow hollow line frame, the recommended ablation site at which the sympathetic nerve is not dominant is marked with a gray hollow line frame, and the ablated site is marked with solid red dots. Optionally, the marker color information corresponds to the ablation effect index, and different ablation effects are represented by different color depths. The above-mentioned ablation prompt information of different marker types may include the ablation prompt information of the ablated site marker, the recommended ablation site marker, or the unrecommended ablation site marker. Of course, it may also include the ablation prompt information of other types of markers. The ablation prompt information of different marker types can be set according to the user requirements, and will not be limited here.

Specifically, as shown in FIG. 4, the dotted frame part indicates a region in which the three-dimensional geometric modeling can be performed, including a right kidney 401. R01 to R04 are ablation site markers in the right renal artery 403 in the three-dimensional geometric model displayed after the electrode stimulation or the ablation of the medical catheter 303. L01 to L02 are ablation site markers in the left renal artery 404 in the three-dimensional geometric model displayed after the electrode stimulation or the ablation of the medical catheter 303. M01 to M02 are ablation site markers in the abdominal aorta 402 in the three-dimensional geometric model displayed after the electrode stimulation or the ablation of the medical catheter 303. In the embodiment, the ablation site markers include a recommended ablation site marker, an unrecommended ablation site marker, and an ablated site marker.

According to th invention, the above-mentioned ablation system further includes an ablation effect determination module configured to calculate an ablation effect index. The ablation effect index depends on ablation parameters. The ablation parameters include an ablation power, a loss power, a surface area of the ablation electrode, and an ablation duration.

Specifically, the ablation effect determination module is mainly configured to calculate the ablation effect index. The ablation effect index is configured to determine whether the ablation of the ablation site is effective according to the parameters and an algorithm, which depends on the ablation parameters. The ablation parameters include the ablation power, the loss power, the surface area of the ablation electrode, and the ablation duration. The ablation power refers to an output power of the ablation energy module. The loss power refers to energy taken away by blood flow and cold saline infusion per unit time. The surface region of the ablation electrode is obtained according to an electrode shape and effective surface region information entered in advance. The electrode shape is generally described with an outer surface size, such as a ring electrode with an outer diameter of 2.0 mm, a width of 1.5 mm, and a surface area of 9.4 mm². The ablation duration refers to the time from the start of the energy output to the end of the energy output of the ablation energy module at the ablation site. Sampling points represent data records of the ablation parameter. One sampling point corresponds to one data record. The number of sampling points refers to the total number of sampling points for one continuous ablation, which is equal to the ablation duration divided by a sampling time interval, for example, the continuous ablation duration of a certain ablation site is 60S, the data sampling time interval is 10mS, then the total number of sampling points is N=6000.

In some embodiments, the ablation effect index can be calculated by the following formula: $AE = {\sum }_{n = 0}^{N} k \frac{\left[Prf\left(n\right)-Pco\left(n\right)\right]}{S} Δ t .$
where AE represents the ablation effect index; *n* denotes the n-th sampling point; *N* denotes the total number of sampling points for one continuous ablation, which is equal to a quotient of the ablation duration divided by a sampling time interval; k is a proportion coefficient ranged from 0.3 to 0.9; Prf(n) represents the ablation power recorded at the n-th sampling point; S represents the surface area of the ablation electrode; Δt is the sampling time interval; Pco(n) represents the loss power corresponding to the n-th sampling point, that is the energy taken away by the blood flow and cold saline infusion per unit time, which is determined by the surface area of the ablation electrode, the temperature and the human body temperature, and blood heat transfer coefficient, or determined by the cold saline temperature, the human body temperature, and saline heat transfer coefficient. Accordingly, Pco(*n*)=*hS*[*T*(*n*)-*TS*], where *h* is a heat exchange coefficient, which can be selected based on experiences, and ranges from 200 to 3000 in W/m²*°C; *T*(*n*) denotes the electrode temperature at the n-th sampling point; *TS* refers to the temperature of the internal environment of the human body, which is the actual body temperature measured under conditions such as non-ablation and non-infusion modes, etc., and which can be a fixed value, such as 37°C, or an average value of the body temperatures measured within a period of time. The units of *T*(*n*) and *TS* are both °C.

In the above embodiment, the ablation effect index is calculated to determine whether the ablation of the ablation site is effective, and the ablation effect index is represented by different colors for users to view.

In some embodiments, the ablation prompt information may further include ablation record information. The information processing module is further configured to receive an instruction for retrieving the information at the ablation site marker. When the instruction is activated, the information processing module displays the ablation record information on the display module. Specifically, by touching, pressing, or clicking the ablation site marker in the corresponding window on the user interface of the display module, the information processing module receives the touching, pressing, or clicking instruction, and retrieves the information at the ablation site marker, and displays the ablation record information on the display module.

The ablation record information may include at least one of the following: an ablation site identifier, spatial position information of an ablation site, a sequence of an ablation site, an ablation power, an ablation current, an intensity of a stimulation signal, a frequency of a stimulation signal, time of a stimulation signal, a physiological parameter change, a temperature, ablation duration, an impedance, an ablation recommendation degree, or an ablation effect index.

The ablation site identifier can be set according to a predefined rule, and the spatial position information of the ablation site is acquired through the input and output module. The sequence of the ablation site is a sequence number of the ablation site, which may further indicate the mark time. Furthermore, the parameters collected in real time, such as the ablation power, the ablation current, the stimulation signal intensity, the impedance, and the temperature, etc., may be displayed through data. For example, these parameters can be displayed either in graphical form or in numerical form for ease of observation.

The ablation effect index provides a more in-depth analysis of the ablation parameters by considering the electrode shape, the effective surface area information entered in advance, the real-time infusion flow and other parameters The specific ablation record information is shown in the following table, which is the record information corresponding to a certain ablation site.

| Site markers | Lxx or Rxx (the xx-th site on Left/Right) | Spatial position information | (x, y, z) or (x, y, z, α, β, γ) |
|---|---|---|---|
| Sequence | The N-th site, record mark time | Ablation power | Value of ablation power |
| Stimulation intensity | Value of voltage or current | Ablation current | Value of ablation current |
| Stimulation frequency | xx times/minute | Impedance | Impedance value during ablation |
| Stimulation time | xx seconds (start time, end time) | Temperature | Temperature value during ablation |
| Blood pressure change | xx seconds before stimulation, xx seconds after stimulation ends | Ablation duration | Start time, end time, time of duration |
| ablation recommendation degree | 1, 2, 3, 4, 5 | Ablation effect (AE) index | AE index is configured to determine effectiveness according to parameters and algorithm |

In the above embodiment, in the three-dimensional geometric model, when a certain marked site is selected, the information corresponding to the site can be displayed, such as the stimulation information, the ablation recommendation degree information, the ablation-related information, etc. Meanwhile, the distribution direction of the arterial nerves can be determined according to the position distribution of existing ablation site markers, to help detect new ablation sites and avoid missing ablation of the arterial nerves. In addition, since the above-mentioned ablation record information includes multi-parameter detection and graphical display involving the ablation energy, time, temperature, impedance, blood pressure, ablation effect which are graphically displayed and combined with the three-dimensional geometric model, the information display is more intuitive, and it is more convenient for the ablation operation, thereby avoiding the repeated ablation and missed ablation target sites, facilitating the search for effective ablation target sites, and improving the safety and effectiveness of the ablation process.

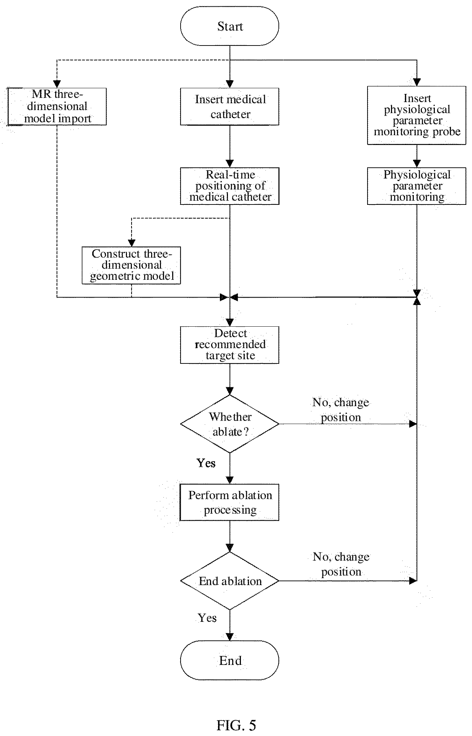

Specifically, as shown in FIG. 5, which is a workflow chart of an ablation system in an embodiment. In the embodiment, the ablation of the renal artery is taken as an example. The device is installed first, and the excitation field generator 200 is installed under the hospital bed, as shown in the dotted box in FIG. 2, the medical catheter 303 is in communication with the ablation instrument 300, and the medical catheter 303 is connected to the signal receiving unit 103 of the three-dimensional mapping system 100. The signal control unit 102 of the three-dimensional mapping system 100 is connected to the excitation field generator 200, and a neutral electrode 302 serves as an energy circuit and is in communication with the ablation instrument 300. If invasive blood pressure monitoring is adopted, the radial artery puncture is selected, the puncture point is at the same level as the heart and is well fixed, and the real-time blood pressure monitoring is started and the blood pressure is recorded.

During the ablation, the information of the medical catheter 303 is generally selected and set first, such as the type of the medical catheter 303 and the parameter of the ablation electrode. Optionally, a three-dimensional image including the renal artery and peripheral arterial vascular tissue is imported into the system, such as a CT image or an MRI image, that is, the three-dimensional image is imported into the information processing module, and the three-dimensional vascular structures of the renal artery and abdominal aorta 402 are segmented through image processing. After that, the medical catheter 303 is inserted into the blood vessel through the incised skin tissue and reaches the abdominal aorta 402 and renal artery regions along the vascular lumen. Other equipment is required to assist in the process. The head end of the medical catheter 303 reaches an effective range for three-dimensional space positioning, that is, the effective working range of the excitation field generator 200. The distal end of the medical catheter 303 can be spatially positioned in real time, and the corresponding spatial position information including a three-dimensional coordinate, a direction, an angle, etc., can be obtained. Meanwhile, an invasive blood pressure probe can be inserted into an appropriate position in the blood vessel of the patient, such as the radial artery, to start the blood pressure monitoring. After the distal end of the medical catheter 303 reaches the effective range, the coordinate obtained in real time can be utilized to perform the three-dimensional modeling of the blood vessels in the corresponding region, to obtain a more intuitive three-dimensional geometric model of the blood vessels.

In such a manner, the step of operating the medical catheter 303 to allow the electrode at the distal end of the medical catheter 303 to search for the target ablation site in the renal artery blood vessel and the nearby abdominal aorta 402 blood vessel regions may include: the physiological stimulation module generates a stimulation signal according to a stimulation parameter, so that the medical catheter 303 outputs the stimulation energy, and the physiological parameter detection module detects a physiological parameter generated in response to the stimulation signal. In this way, the signal input and output module acquires the spatial position information of the stimulated site according to the current position of the medical catheter 303, and transmits the spatial position information of the stimulated site to the information processing module. The information processing module generates ablation prompt information according to the physiological parameter and the spatial position information of the stimulated site. The ablation prompt information includes recommended ablation site information and unrecommended ablation site information. Optionally, the recommended ablation site information and unrecommended ablation site information are displayed on the three-dimensional geometric model. That is to say, the information processing module records the identifiers and parameters for finding the target ablation sites, and associates them with the corresponding mark points in the three-dimensional geometric model for easy viewing. Furthermore, according to the recommendation result of the recommended ablation site, the current recommended ablation site can serve as the target ablation site, and ablation is started at the recommended ablation site, and the ablation-related information is recorded at the same time, such as the power, temperature, impedance, and cold saline infusion flow rate. The ablation effect quantification index is calculated according to the ablation parameter, and combined with the response of other physiological parameters of the patient, it is determined whether the recommended ablation site achieves the purpose. If the purpose is achieved, the ablation at the recommended ablation site can be stopped, otherwise, the ablation can be continued. After denervation ablation of multiple ablation target sites for the sympathetic nerves, it is determined whether the ablation purpose is achieved. If the ablation target is achieved, the ablation can be stopped, otherwise, other ablation target sites can be searched for to continue the ablation until the ultimate purpose is achieved.

In the above embodiment, the medical catheter 303 can be utilized to realize the three-dimensional spatial positioning of the distal end of the medical catheter 303 in the renal artery, and to model the vascular lumen structures of the bilateral renal arteries and/or the abdominal aorta 402 segment according to the positioning of the distal end of the medical catheter 303. The combination of three-dimensional spatial positioning and modeling technology and the radiofrequency ablation technology upgrades the conventional ablation processing of the renal artery from a two-dimensional mode of fuzzy positioning to a three-dimensional mode capable of performing the three-dimensional modeling and the precise positioning, thereby significantly reducing or even completely avoid the use of X-ray imaging, reducing the impact on the health of patients and doctors. Furthermore, the real-time positioning of the medical catheter 303 can be realized, seamless communication of the data information can be realized, and the coordinate information of each operated ablation site and relevant technical parameters for performing the ablation operation can be displayed.

Further, it should be noted that the above embodiments of the present invention are illustrated by taking the hardware structures shown in FIGS. 2 and 3 as examples, but the hardware structure of the ablation system in the present invention is not limited to that shown in FIGS. 2 and 3. For example, the information processing module in the present invention may include the working module 101 in FIG. 2 and the intelligent analysis module in FIG. 3. In other embodiments, the information processing module may include only one of the working module 101 in FIG. 2 and the intelligent analysis module in FIG. 3, and the functions of the work module 101 and the intelligent analysis module are pre-configured into this one as required. The display module in the present invention can be as shown in FIGS. 2 and 3, which is only the human-machine interaction module in FIG. 3. In other embodiments, there may exist two display modules in the present invention, one of which is located in the three-dimensional mapping system 100 in FIG. 2, and the other display module is located in the radiofrequency ablation instrument 300 in FIG. 2. The contents displayed by these two display modules can be pre-configured as required, which are not specifically limited here.

It should be appreciated that although various steps in the flow chart of FIG. 5 are shown in sequence as indicated by arrows, these steps are not definitely executed in the order indicated by the arrows. Unless explicitly stated in this article, there is no strict order restriction on the execution of these steps, and these steps can be executed in other orders. Moreover, at least some of the steps in FIG. 5 may include multiple steps or stages. These steps or stages are not definitely executed at the same time, but may be executed at different time. These steps or stages are not definitely performed in sequence, but may be performed in turn or alternately with other steps or at least part of steps or stages in other steps. Those of ordinary skill in the art can understand that all or part of the processes in the methods of the above embodiments can be implemented by instructing relevant hardware through computer-readable instructions. The computer-readable instructions can be stored in a non-transitory computer-readable storage medium or a transitory computer-readable storage medium. When the computer-readable instructions are executed, the processes of the embodiments of the above methods can be included. Any reference to the memory, storage, database or other media used in the embodiments provided in the present invention may include at least one of a non-transitory memory and a transitory memory. The non-transitory memory may include a read-only memory (ROM), a magnetic tape, a floppy disk, a flash memory or an optical memory, etc. The transitory memory may include a random access memory (RAM) or an external cache memory. By way of illustration but not limitation, the RAM can be in various forms, such as static random access memory (SRAM) or dynamic random access memory (DRAM), etc. The technical features in the above embodiments can be combined in any way. To simplify the description, not all possible combinations of the technical features in the above embodiments are described. Moreover, although the innovation of the present invention comes from the renal artery ablation, those skilled in the art can understand that the present invention can also be applied to ablation at different sites such as cardiac ablation and bronchial ablation, etc. In addition, the radiofrequency ablation energy is listed in the embodiments of the present invention. Other ablation energies such as pulse ablation and microwave ablation, etc., may also be used in the present invention, which is not limited here. The above-mentioned embodiments only express several implementation modes of the present invention, and descriptions thereof are relatively specific and detailed. Therefore, the protection scope of the present invention should be subject to the appended claims.

## Claims

1. An ablation system, comprising an information processing module and a signal input and output module in communication with each other, wherein
the signal input and output module is configured to be connected to at least one medical catheter, and acquire spatial position information of an ablation site according to a current position of the medical catheter when the medical catheter outputs ablation energy, and transmit the spatial position information of the ablation site to the information processing module; and
the information processing module is configured to acquire target spatial position information according to a target ablation site, periodically calculate and obtain to-be-compared position information according to the spatial position information of the ablation site within a predetermined time period which includes obtaining the to-be-compared position information by calculating statistical information of the spatial position information of all ablation sites within the predetermined time period, and periodically compare the to-be-compared position information to the target spatial position information to generate ablation prompt information;
wherein the signal input and output module is further configured to acquire modeling spatial position information according to spatial position information of a distal end of the medical catheter (303) and transmit the modeling spatial position information to the information processing module, and the information processing module is further configured to construct a three-dimensional geometric model of a region in which the distal end of the medical catheter is located according to the modeling spatial position information and mark the generated ablation prompt information into the three-dimensional geometric model;
the ablation system further comprising:
an ablation energy module, connected to the medical catheter, and configured to generate an ablation signal according to ablation parameters, to allow the medical catheter to output the ablation energy; and
a display module, in communication with the information processing module, and configured to display the three-dimensional geometric model and the ablation prompt information;
wherein the ablation prompt information comprises marker color information, and ablation prompt information of different marker types corresponds to different marker color information;
**characterized in that**
the ablation system further comprising an ablation effect determination module configured to calculate an ablation effect index, wherein the ablation effect index depends on ablation parameters comprising an ablation power, a loss power, a surface area of an ablation electrode, and an ablation duration;
wherein the marker color information corresponds to the ablation effect index, and different ablation effects are represented by different color depths.

2. The system according to claim 1, wherein the information processing module is further configured to record the target spatial position information as spatial position information of an ablated site when the medical catheter completes ablation if a distance calculated according to the current to-be-compared position information within the current predetermined time period and the target spatial position information is less than or equal to a first preset distance.

3. The system according to claim 2, wherein the information processing module is further configured to output a prompt indicating that an ablation position moves when the distance calculated according to the current to-be-compared position information within the current predetermined time period and the target spatial position information is greater than the first preset distance.

4. The system according to claim 2, wherein the information processing module is further configured to compare the current to-be-compared position information within the current predetermined time period to spatial position information of a previously ablated site, and output a prompt indicating repeated ablation when a distance calculated according to the current to-be-compared position information within the current predetermined time period and the spatial position information of the previously ablated site is less than a second preset distance.

5. The system according to claim 2, further comprising:
a physiological stimulation module, configured to be connected to the medical catheter, and configured to generate a stimulation signal according to a stimulation parameter, to allow the medical catheter to output stimulation energy; and
a physiological parameter detection module, configured to detect a physiological parameter generated in response to the stimulation signal, and transmit the physiological parameter to the information processing module, wherein
when the medical catheter outputs the stimulation energy, the information input and output module is configured to acquire spatial position information of a stimulation site according to the current position of the medical catheter and transmit the spatial position information of the stimulation site to the information processing module, the information processing module is further configured to generate the ablation prompt information according to the physiological parameter and the spatial position information of the stimulation site, wherein the ablation prompt information comprises recommended ablation site information where stimulation site information satisfying an ablation requirement and unrecommended ablation site information.

6. The system according to claim 5, wherein the information processing module is further configured to determine the spatial position information of the corresponding stimulation site in the recommended ablation site information as the target spatial position information of the target ablation site, calculate a distance between the target spatial position information of the current target ablation site and spatial position information of a previously ablated site, and output a prompt indicating repeated ablation when the distance is less than or equal to a third preset distance.

7. The system according to claim 5, wherein the information processing module is further configured to determine the spatial position information of the corresponding stimulation site in the recommended ablation site information as the target spatial position information of the target ablation site, calculate a distance between the target spatial position information of the current target ablation site and the to-be-compared position information, and output a prompt indicating that the ablation position moves when the distance is greater than a fourth preset distance.

8. The system according to claim 1, wherein the ablation effect index is calculated by the following formula: $AE = {\sum }_{n = 0}^{N} k \frac{\left[Prf\left(n\right)-Pco\left(n\right)\right]}{S} Δ t ,$ where AE represents the ablation effect index, *n* denotes the n-th sampling point, *N* denotes the total number of sampling points for one continuous ablation which is equal to a quotient of the ablation duration divided by a sampling time interval, k is a proportion coefficient ranged from 0.3 to 0.9, Prf(n) represents the ablation power recorded at the n-th sampling point, S represents the surface area of the ablation electrode, Δt is the sampling time interval, Pco(n) represents the loss power corresponding to the n-th sampling point, Pco(*n*)*=hS*[*T*(*n*)*-TS*]*,* wherein *h* is a heat exchange coefficient and is ranged from 200 to 3000 in W/m²*°C, *T*(*n*) denotes an electrode temperature at the n-th sampling point, TS represents a temperature of an internal environment of a human body with a unit °C.

9. The system according to claim 1, wherein the ablation prompt information further comprises ablation record information, and the information processing module is further configured to receive an instruction for retrieving information at an ablation site marker, and display the ablation record information on the display module when the instruction is activated.

10. The system according to claim 9, wherein the ablation record information comprises at least one of the following: an ablation site identifier, spatial position information of an ablation site, a sequence of an ablation site, an ablation power, an ablation current, an intensity of a stimulation signal, a frequency of a stimulation signal, time of a stimulation signal, a physiological parameter change, a temperature, ablation duration, an impedance, an ablation recommendation degree, or the ablation effect index.

11. The system according to claim 1, wherein the three-dimensional geometric model comprises renal artery vessels and/or abdominal aorta segment vessels.

## Patentansprüche

1. Ablationssystem, umfassend ein Informationsverarbeitungsmodul und ein Signaleingabe- und -ausgabemodul, die miteinander in Kommunikation stehen, wobei
das Signaleingabe- und -ausgabemodul konfiguriert ist, um mit mindestens einem medizinischen Katheter verbunden zu sein und Raumpositionsinformationen einer Ablationsstelle gemäß einer aktuellen Position des medizinischen Katheters zu erfassen, wenn der medizinische Katheter Ablationsenergie ausgibt, und die Raumpositionsinformationen der Ablationsstelle an das Informationsverarbeitungsmodul zu übertragen; und
das Informationsverarbeitungsmodul konfiguriert ist, um Zielraumpositionsinformationen gemäß einer Zielablationsstelle zu erfassen, zu vergleichende Positionsinformationen gemäß den Zielraumpositionsinformationen der Ablationsstelle innerhalb einer vorbestimmten Zeitperiode periodisch zu berechnen und zu erhalten, was das Erhalten der zu vergleichenden Positionsinformationen durch Berechnen statistischer Informationen der Raumpositionsinformationen aller Ablationsstellen innerhalb der vorbestimmten Zeitperiode einschließt, und die zu vergleichenden Positionsinformationen mit den Zielraumpositionsinformationen periodisch zu vergleichen, um Ablationsaufforderungsinformationen zu erzeugen;
wobei das Signaleingabe- und -ausgabemodul ferner konfiguriert ist, um Modellierungsraumpositionsinformationen gemäß Raumpositionsinformationen eines distalen Endes des medizinischen Katheters (303) zu erfassen und die Modellierungsraumpositionsinformationen an das Informationsverarbeitungsmodul zu übertragen, und das Informationsverarbeitungsmodul ferner konfiguriert ist, um ein dreidimensionales geometrisches Modell eines Bereichs, in dem sich das distale Ende des medizinischen Katheters befindet, gemäß den Modellierungsraumpositionsinformationen zu konstruieren und die erzeugten Ablationsaufforderungsinformationen in dem dreidimensionalen geometrischen Modell zu markieren;
das Ablationssystem ferner umfassend:
ein Ablationsenergiemodul, das mit dem medizinischen Katheter verbunden und konfiguriert ist, um ein Ablationssignal gemäß Ablationsparametern zu erzeugen, um dem medizinischen Katheter zu ermöglichen, die Ablationsenergie auszugeben; und
ein Anzeigemodul, das mit dem Informationsverarbeitungsmodul in Kommunikation steht und konfiguriert ist, um das dreidimensionale geometrische Modell und die Ablationsaufforderungsinformationen anzuzeigen;
wobei die Ablationsaufforderungsinformationen Markierungsfarbinformationen umfassen und Ablationsaufforderungsinformationen unterschiedlicher Markierungsarten unterschiedlichen Markierungsfarbinformationen entsprechen;
**dadurch gekennzeichnet, dass** das Ablationssystem ferner ein Ablationseffektbestimmungsmodul umfasst, das konfiguriert ist, um einen Ablationseffektindex zu berechnen, wobei der Ablationseffektindex von Ablationsparametern, umfassend eine Ablationsleistung, eine Verlustleistung, einen Oberflächeninhalt einer Ablationselektrode und eine Ablationsdauer, abhängt;
wobei die Markierungsfarbinformationen dem Ablationseffektindex entsprechen und unterschiedliche Ablationseffekte durch unterschiedliche Farbtiefen dargestellt sind.

2. System nach Anspruch 1, wobei das Informationsverarbeitungsmodul ferner konfiguriert ist, um die Zielraumpositionsinformationen als Raumpositionsinformationen einer ablatierten Stelle aufzuzeichnen, wenn der medizinische Katheter die Ablation fertigstellt, falls ein Abstand, der gemäß den aktuellen zu vergleichenden Positionsinformationen innerhalb der aktuellen vorbestimmten Zeitperiode und den Zielraumpositionsinformationen berechnet wird, kleiner als oder gleich einem ersten voreingestellten Abstand ist.

3. System nach Anspruch 2, wobei das Informationsverarbeitungsmodul ferner konfiguriert ist, um eine Aufforderung auszugeben, die angibt, dass sich eine Ablationsposition bewegt, wenn der Abstand, der gemäß den aktuellen zu vergleichenden Positionsinformationen innerhalb der aktuellen vorbestimmten Zeitperiode und den Zielraumpositionsinformationen berechnet wird, größer als der erste voreingestellte Abstand ist.

4. System nach Anspruch 2, wobei das Informationsverarbeitungsmodul ferner konfiguriert ist, um die aktuellen zu vergleichenden Positionsinformationen innerhalb der aktuellen vorbestimmten Zeitperiode mit Raumpositionsinformationen einer zuvor ablatierten Stelle zu vergleichen und eine Aufforderung auszugeben, die die wiederholte Ablation angibt, wenn ein Abstand, der gemäß den aktuellen zu vergleichenden Positionsinformationen innerhalb der aktuellen vorbestimmten Zeitperiode und den räumlichen Raumpositionsinformationen der zuvor ablatierten Stelle berechnet wird, kleiner als ein zweiter voreingestellter Abstand ist.

5. Verfahren nach Anspruch 2, ferner umfassend:
ein Modul für physiologische Stimulation, das konfiguriert ist, um mit dem medizinischen Katheter verbunden zu sein, und konfiguriert ist, um ein Stimulationssignal gemäß einem Stimulationsparameter zu erzeugen, um dem medizinischen Katheter zu ermöglichen, Stimulationsenergie auszugeben; und
ein Modul für eine Detektion des physiologischen Parameters, das konfiguriert ist, um einen physiologischen Parameter zu detektieren, der als Reaktion auf das Stimulationssignal erzeugt wird, und den physiologischen Parameter an das Informationsverarbeitungsmodul zu übertragen, wobei
wenn der medizinische Katheter die Stimulationsenergie ausgibt, das Informationseingabe- und -ausgabemodul konfiguriert ist, um Raumpositionsinformationen einer Stimulationsstelle gemäß der aktuellen Position des medizinischen Katheters zu erfassen und die Raumpositionsinformationen der Stimulationsstelle an das Informationsverarbeitungsmodul zu übertragen, wobei das Informationsverarbeitungsmodul ferner konfiguriert ist, um die Ablationsaufforderungsinformationen gemäß dem physiologischen Parameter und den Raumpositionsinformationen der Stimulationsstelle zu erzeugen, wobei die Ablationsaufforderungsinformationen Informationen einer empfohlenen Ablationsstelle, wo Stimulationsstelleninformationen eine Ablationsanforderung erfüllen, und Informationen einer nicht empfohlenen Ablationsstelle umfassen.

6. System nach Anspruch 5, wobei das Informationsverarbeitungsmodul ferner konfiguriert ist, um die Raumpositionsinformationen der entsprechenden Stimulationsstelle in den Informationen der empfohlenen Ablationsstelle als die Zielraumpositionsinformationen der Zielablationsstelle zu bestimmen, einen Abstand zwischen den Zielraumpositionsinformationen der aktuellen Zielablationsstelle und Raumpositionsinformationen einer zuvor ablatierten Stelle zu berechnen und eine Aufforderung auszugeben, die die wiederholte Ablation angibt, wenn der Abstand kleiner als oder gleich einem dritten voreingestellten Abstand ist.

7. System nach Anspruch 5, wobei das Informationsverarbeitungsmodul ferner konfiguriert ist, um die Raumpositionsinformationen der entsprechenden Stimulationsstelle in den Informationen der empfohlenen Ablationsstelle als die Zielraumpositionsinformationen der Zielablationsstelle zu bestimmen, einen Abstand zwischen den Zielraumpositionsinformationen der aktuellen Zielablationsstelle und den zu vergleichenden Positionsinformationen zu berechnen und eine Aufforderung auszugeben, die angibt, dass sich die Ablationsposition bewegt, wenn der Abstand größer als ein vierter voreingestellter Abstand ist.

8. System nach Anspruch 1, wobei der Ablationseffektindex durch die folgende Formel berechnet wird: $AE = {\sum }_{n = 0}^{N} k \frac{\left[Prf\left(n\right)-Pco\left(n\right)\right]}{S} Δ t ,$ wobei AE den Ablationseffektindex darstellt, *n* den *n*-ten Abtastpunkt bezeichnet, *N* die Gesamtanzahl der Abtastpunkte für eine kontinuierliche Ablation bezeichnet, die gleich einem Quotienten der Ablationsdauer dividiert durch ein Abtastzeitintervall ist, *k* ein Proportionskoeffizient in einem Bereich von 0,3 bis 0,9 ist, Prf(*n*) die Ablationsleistung darstellt, die an dem *n*-ten Abtastpunkt aufgezeichnet wird, *S* den Oberflächeninhalt der Ablationselektrode darstellt, Δt das Abtastzeitintervall ist, Pco(*n*) die Verlustleistung darstellt, die dem *n*-ten Abtastpunkt entspricht, Pco(*n*)=*hS*[*T*(*n*)-*TS*], wobei *h* ein Wärmeaustauschkoeffizient ist und in einem Bereich von 200 bis 3000 in W/m²*°C liegt, *T*(*n*) eine Elektrodentemperatur an dem *n*-ten Abtastpunkt bezeichnet, *TS* eine Temperatur einer inneren Umgebung eines menschlichen Körpers mit einer Einheit °C darstellt.

9. System nach Anspruch 1, wobei die Ablationsaufforderungsinformationen ferner Ablationsaufzeichnungsinformationen umfassen und das Informationsverarbeitungsmodul ferner konfiguriert ist, um eine Anweisung zum Abrufen von Informationen an einer Ablationsstellenmarkierung zu empfangen und die Ablationsaufzeichnungsinformationen auf dem Anzeigemodul anzuzeigen, wenn die Anweisung aktiviert ist.

10. System nach Anspruch 9, wobei die Ablationsaufzeichnungsinformationen mindestens eines der Folgenden umfassen: eine Ablationsstellenkennung, Raumpositionsinformationen einer Ablationsstelle, eine Sequenz einer Ablationsstelle, eine Ablationsleistung, einen Ablationsstrom, eine Intensität eines Stimulationssignals, eine Frequenz eines Stimulationssignals, eine Zeit eines Stimulationssignals, eine Änderung des physiologischen Parameters, eine Temperatur, eine Ablationsdauer, eine Impedanz, einen Ablationsempfehlungsgrad oder den Ablationseffektindex.

11. System nach Anspruch 1, wobei das dreidimensionale geometrische Modell Nierenarteriengefäße und/oder Abdominalaortensegmentgefäße umfasst.

## Revendications

1. Système d'ablation, comprenant un module de traitement d'informations et un module d'entrée et de sortie de signal en communication l'un avec l'autre, dans lequel
le module d'entrée et de sortie de signal est configuré pour être connecté à au moins un cathéter médical, et acquérir des informations de position spatiale d'un site d'ablation selon une position actuelle du cathéter médical lorsque le cathéter médical délivre une énergie d'ablation, et transmettre les informations de position spatiale du site d'ablation au module de traitement d'informations ; et
le module de traitement d'informations est configuré pour acquérir des informations de position spatiale cible selon un site d'ablation cible, calculer et obtenir de manière périodique des informations de position à comparer selon les informations de position spatiale du site d'ablation au cours d'une période de temps prédéterminée qui comporte l'obtention des informations de position à comparer en calculant des informations statistiques des informations de position spatiale de tous les sites d'ablation au cours de la période de temps prédéterminée, et comparer de manière périodique les informations de position à comparer aux informations de position spatiale cible pour générer des informations d'invite d'ablation ;
dans lequel le module d'entrée et de sortie de signal est en outre configuré pour acquérir des informations de position spatiale de modélisation selon des informations de position spatiale d'une extrémité distale du cathéter médical (303) et transmettre les informations de position spatiale de modélisation au module de traitement d'informations, et le module de traitement d'informations est en outre configuré pour construire un modèle géométrique tridimensionnel d'une région dans laquelle l'extrémité distale du cathéter médical est située selon les informations de position spatiale de modélisation et marquer les informations d'invite d'ablation générées dans le modèle géométrique tridimensionnel ;
le système d'ablation comprenant en outre :
un module d'énergie d'ablation, connecté au cathéter médical, et configuré pour générer un signal d'ablation selon des paramètres d'ablation, pour permettre au cathéter médical de délivrer l'énergie d'ablation ; et
un module d'affichage, en communication avec le module de traitement d'informations, et configuré pour afficher le modèle géométrique tridimensionnel et les informations d'invite d'ablation ;
dans lequel les informations d'invite d'ablation comprennent des informations de couleur de marqueur, et les informations d'invite d'ablation de différents types de marqueurs correspondent à différentes informations de couleur de marqueur ;
**caractérisé en ce que** le système d'ablation comprend en outre un module de détermination d'effet d'ablation configuré pour calculer un indice d'effet d'ablation, dans lequel l'indice d'effet d'ablation dépend de paramètres d'ablation comprenant une puissance d'ablation, une puissance de perte, une surface d'une électrode d'ablation et une durée d'ablation ;
dans lequel les information de couleur de marqueur correspondent à l'indice d'effet d'ablation, et différents effets d'ablation sont représentés par différentes profondeurs de couleur.

2. Système selon la revendication 1, dans lequel le module de traitement d'informations est en outre configuré pour enregistrer les informations de position spatiale cible en tant qu'informations de position spatiale d'un site ablaté lorsque le cathéter médical termine une ablation si une distance calculée selon les informations de position à comparer actuelles au cours de la période de temps prédéterminée actuelle et les informations de position spatiale cible est inférieure ou égale à une première distance prédéfinie.

3. Système selon la revendication 2, dans lequel le module de traitement d'informations est en outre configuré pour délivrer une invite indiquant qu'une position d'ablation se déplace lorsque la distance calculée selon les informations de position à comparer actuelles au cours de la période de temps prédéterminée actuelle et les informations de position spatiale cible est supérieure à la première distance prédéfinie.

4. Système selon la revendication 2, dans lequel le module de traitement d'informations est en outre configuré pour comparer les informations de position à comparer actuelles au cours de la période de temps prédéterminée actuelle à des informations de position spatiale d'un site préalablement ablaté, et délivrer une invite indiquant une ablation répétée lorsqu'une distance calculée selon les informations de position à comparer actuelles au cours de la période de temps prédéterminée actuelle et les informations de position spatiale du site préalablement ablaté est inférieure à une deuxième distance prédéfinie.

5. Système selon la revendication 2, comprenant en outre :
un module de stimulation physiologique, configuré pour être connecté au cathéter médical, et configuré pour générer un signal de stimulation selon un paramètre de stimulation, pour permettre au cathéter médical de délivrer une énergie de stimulation ; et
un module de détection de paramètre physiologique, configuré pour détecter un paramètre physiologique généré en réponse au signal de stimulation, et transmettre le paramètre physiologique au module de traitement d'informations, dans lequel
lorsque le cathéter médical délivre l'énergie de stimulation, le module d'entrée et de sortie d'informations est configuré pour acquérir des informations de position spatiale d'un site de stimulation selon la position actuelle du cathéter médical et transmettre les informations de position spatiale du site de stimulation au module de traitement d'informations, le module de traitement d'informations est en outre configuré pour générer les informations d'invite d'ablation selon le paramètre physiologique et les informations de position spatiale du site de stimulation, dans lequel les informations d'invite d'ablation comprennent des informations de site d'ablation recommandé où des informations de site de stimulation satisfont une exigence d'ablation et des informations de site d'ablation non recommandé.

6. Système selon la revendication 5, dans lequel le module de traitement d'informations est en outre configuré pour déterminer les informations de position spatiale du site de stimulation correspondant dans les informations de site d'ablation recommandé en tant qu'informations de position spatiale cible du site d'ablation cible, calculer une distance entre les informations de position spatiale cible du site d'ablation cible actuel et des informations de position spatiale d'un site préalablement ablaté, et délivrer une invite indiquant une ablation répétée lorsque la distance est inférieure ou égale à une troisième distance prédéfinie.

7. Système selon la revendication 5, dans lequel le module de traitement d'informations est en outre configuré pour déterminer les informations de position spatiale du site de stimulation correspondant dans les informations de site d'ablation recommandé en tant qu'informations de position spatiale cible du site d'ablation cible, calculer une distance entre les informations de position spatiale cible du site d'ablation cible actuel et les informations de position à comparer, et délivrer une invite indiquant que la position d'ablation se déplace lorsque la distance est supérieure à une quatrième distance prédéfinie.

8. Système selon la revendication 1, dans lequel l'indice d'effet d'ablation est calculé par la formule suivante : $AE = {\sum }_{n = 0}^{N} k \frac{\left[Prf\left(n\right)-Pco\left(n\right)\right]}{S} Δ t ,$ où AE représente l'indice d'effet d'ablation, *n* désigne le *n*-ième point d'échantillonnage, *N* désigne le nombre total de points d'échantillonnage pour une ablation continue qui est égal à un quotient de la durée d'ablation divisée par un intervalle de temps d'échantillonnage, *k* est un coefficient de proportion compris entre 0,3 et 0,9, Prf(*n*) représente la puissance d'ablation enregistrée au niveau du *n*-ième point d'échantillonnage, *S* représente la surface de l'électrode d'ablation, Δt est l'intervalle de temps d'échantillonnage, Pco(*n*) représente la puissance de perte correspondant au *n*-ième point d'échantillonnage, Pco(*n*) = *hS*[*T*(*n*) - *TS*], où *h* est un coefficient d'échange thermique et est compris entre 200 et 3 000 en W/m² * °C, *T*(*n*) désigne une température d'électrode au niveau du *n*-ième point d'échantillonnage, *TS* représente une température d'un environnement interne d'un corps humain avec une unité °C.

9. Système selon la revendication 1, dans lequel les informations d'invite d'ablation comprennent en outre des informations d'enregistrement d'ablation, et le module de traitement d'informations est en outre configuré pour recevoir une instruction pour récupérer des informations au niveau d'un marqueur de site d'ablation, et afficher les informations d'enregistrement d'ablation sur le module d'affichage lorsque l'instruction est activée.

10. Système selon la revendication 9, dans lequel les informations d'enregistrement d'ablation comprennent au moins l'un parmi les éléments suivants : un identifiant de site d'ablation, des informations de position spatiale d'un site d'ablation, une séquence d'un site d'ablation, une puissance d'ablation, un courant d'ablation, une intensité d'un signal de stimulation, une fréquence d'un signal de stimulation, une heure d'un signal de stimulation, un changement de paramètre physiologique, une température, une durée d'ablation, une impédance, un degré de recommandation d'ablation, ou l'indice d'effet d'ablation.

11. Système selon la revendication 1, dans lequel le modèle géométrique tridimensionnel comprend des vaisseaux d'artère rénale et/ou des vaisseaux de segment d'aorte abdominale.
